# FASCICULE DE BREVET EUROPEEN

(11) **EP 3 551 250 B1**
(45) Date de publication et mention de la délivrance du brevet: **23.10.2024**
(21) Numéro de dépôt: 17809321.7
(22) Date de dépôt: 07.12.2017
(51) Int. Cl.: A61M 5/142

(54) **DISPOSITIF IMPLANTABLE**
IMPLANTIERBARE VORRICHTUNG
IMPLANTABLE DEVICE

(30) Priorité: 07.12.2016 FR 1662064
(43) Date de publication de la demande: 16.10.2019
(73) Titulaire: UNIVERSITE GRENOBLE ALPES, 38400 Saint Martin d'Hères (FR); Centre Hospitalier Universitaire Grenoble Alpes, 38700 La Tronche (FR)
(72) Inventeur: CINQUIN, Philippe, 38330 Saint Nazaire les Eymes (FR); TUVIGNON, Patrick, 81000 Albi (FR)
(74) Mandataire: Hautier IP
(86) Numéro de dépôt international: PCT/EP2017/081905
(87) Numéro de publication internationale: WO 2018/104481

(56) Documents cités:
- EP-A2- 1 504 778
- WO-A1-2011/150032
- WO-A2-02/087657
- WO-A2-2006/035446
- US-A1- 2004 147 871

## Description

La présente invention concerne un dispositif implantable. La présente invention concerne également un procédé d'administration d'une substance active à un patient.

Des dispositifs implantés dans le corps sont utilisés pour administrer des médicaments à des patients. Les dispositifs implantés comprennent alors une batterie et une réserve de médicament. Cependant, il est nécessaire de recharger ou de remplacer régulièrement les batteries et les réserves de tels dispositifs implantés. En particulier, dans de nombreux cas ce remplacement est effectué par une opération chirurgicale. Une telle procédure est relativement chère et contraignante pour le patient puisqu'elle a lieu dans un bloc opératoire d'un établissement hospitalier et qu'une anesthésie est nécessaire, ainsi qu'un séjour prolongé dans l'établissement hospitalier afin de surveiller les suites de l'opération. En outre, comme pour toute intervention chirurgicale, il existe des risques que le patient contracte une infection lors de l'opération.

Dans d'autres cas, des dispositifs implantés du type précité sont alimentés depuis l'extérieur par un module de stockage d'énergie qui est porté par le patient à l'extérieur de son corps. Par exemple, il arrive que des dispositifs d'alimentation transmettent de l'énergie par ondes ultrasonores au dispositif de stimulation, à travers la peau et la cage thoracique du patient. Cependant, les ondes ultrasonores traversent mal les os, et une grande précision dans le placement de la source d'ultra-sons est alors nécessaire, dans les cas où le dispositif implanté est situé devant la cage thoracique, afin d'assurer une bonne alimentation du dispositif implanté. En outre, un tel dispositif d'alimentation extérieur au corps du patient est disgracieux.

Il arrive également que des dispositifs implantables soient équipés de connecteurs filaires, permettant une connexion électrique ou un transfert de fluide entre le dispositif implantable et un dispositif extérieur. Par ce biais, un courant électrique d'alimentation ou un flux de médicament sont échangés avec le dispositif extérieur. Là encore, ces connecteurs débouchant à travers la peau du patient sont disgracieux, et présentent nécessairement des risques sanitaires ainsi que des contraintes importantes pour le patient dans sa vie de tous les jours.

Il existe donc un besoin pour un système implantable qui soit moins contraignant pour le patient.

Il est connu du document WO 02/087657 A2 un dispositif implantable pour le traitement de l'estomac, configuré pour délivrer une substance active par un conduit à l'intérieur de l'estomac, dans sa paroi ou à l'extérieur de la paroi de l'estomac. Le
document EP 1504 778 A2 divulgue un dispositif de délivrance de substance active dans le tractus gastro-intestinal, visant à induire la satiété chez un patient. Le document WO 2006/035446 A2 divulgue un dispositif implantable pour détecter un changement physiologique associé à l'ingestion d'aliments ou à la faim, et comprenant un mécanisme adapté pour stimuler directement une région par une substance active propre à déclencher la satiété.

Il est proposé un dispositif implantable propre à être fixé dans une position de fixation à une paroi de l'estomac d'un patient, le dispositif implantable étant accueilli dans l'estomac lorsque le dispositif implantable est dans la position de fixation, le dispositif implantable comprenant une réserve d'une substance active et un injecteur propre à administrer la substance active au patient, le dispositif implantable étant caractérisé en ce que l'injecteur comporte deux cathéters, l'injecteur étant configuré pour administrer la substance active dans un fluide corporel du patient à travers au moins un des cathéters, l'injecteur étant configuré pour aspirer, par l'un des cathéters le fluide corporel du patient et pour rejeter par l'autre cathéter le fluide corporel aspiré.

Selon des modes de réalisation, le dispositif implantable comprend l'une ou plusieurs des caractéristiques suivantes, prises isolément ou suivant toutes les combinaisons techniquement possibles :
- l'injecteur est configuré pour injecter la substance active dans un fluide corporel du patient ;
- le fluide corporel est le liquide péritonéal ou le sang ;
- l'injecteur est configuré pour injecter la substance active dans le tube digestif du patient ;
- l'injecteur comporte au moins un cathéter propre à permettre l'injection de la substance active, par l'injecteur, dans une lumière de l'intestin du patient ;
- la substance active est choisie parmi l'ensemble formé de l'insuline, la levodopa, un antidouleur et un anticancéreux ;
- le dispositif implantable est propre à être fixé dans la partie supérieure de l'estomac ;
- la réserve de substance active comprend un connecteur solidaire de l'injecteur et une capsule contenant la substance active, la capsule étant propre à être avalée par le patient et à venir se connecter au connecteur pour délivrer la substance active à l'injecteur ;
- le dispositif implantable comprend un capteur et au moins un cathéter propre à conduire un fluide corporel du patient au capteur, le capteur étant propre à mesurer au moins une valeur d'un taux d'un marqueur biologique dans le fluide corporel ;
- le dispositif implantable comprend en outre un contrôleur propre à commander l'administration, par l'injecteur, de la substance active en fonction d'au moins une valeur du taux mesurée ;
- le contrôleur est propre à calculer une fréquence et/ou une dose de substance active à administrer au patient en fonction d'au moins une valeur du taux mesurée ;
- le fluide corporel est le liquide péritonéal ou le sang ;
- le dispositif implantable comprend une alimentation électrique comportant une réserve d'énergie électrique amovible et un connecteur propre à accueillir la réserve d'énergie électrique, la réserve d'énergie électrique étant propre à alimenter électriquement l'injecteur lorsque la réserve d'énergie électrique est connectée électriquement au connecteur dans une position de connexion et de préférence étant configurée pour être avalée par le patient et pour se déplacer spontanément jusqu'à la position de connexion depuis une position de déconnexion dans laquelle la réserve d'énergie électrique est accueillie dans l'estomac du patient et est déconnectée du connecteur ;
- le dispositif implantable comprend une alimentation électrique propre à générer un courant électrique d'alimentation de l'injecteur par réaction d'au moins une espèce chimique présente dans le corps du patient, notamment le glucose ;
- le dispositif implantable comprend une alimentation électrique propre à générer un courant électrique d'alimentation de l'injecteur par conversion d'énergie mécanique en énergie électrique.

Le dispositif implantable comprend deux premiers cathéters. Les deux premiers cathéters peuvent être raccordés, par l'intermédiaire de la pompe, pour former une continuité fluidique. Un premier cathéter peut être relié à l'aspiration de la pompe, et le deuxième au refoulement. Aspiration et refoulement du liquide corporel sont assurés par la pompe.

Des caractéristiques et avantages de l'invention apparaîtront à la lecture de la description qui va suivre, donnée uniquement à titre d'exemple non limitatif et faite en référence aux dessins annexés, sur lesquels :
- la figure 1 est un schéma d'un exemple de système implantable comportant une alimentation électrique,
- la figure 2 est une représentation schématique du dispositif implantable de la figure 1, implanté dans le corps d'un patient, et
- la figure 3 est une représentation schématique de l'alimentation de la figure 1.

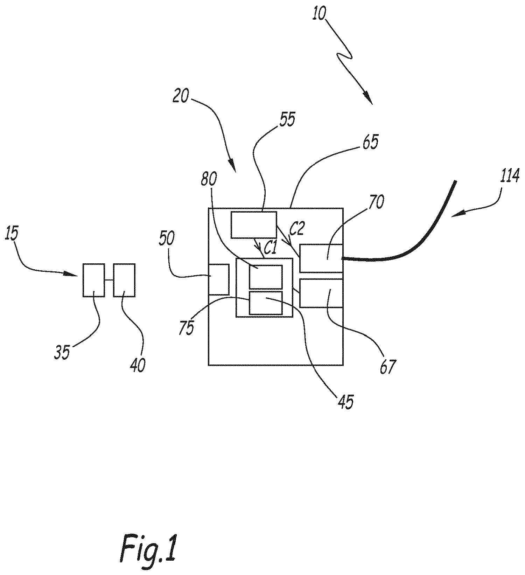

Un premier exemple de système implantable 10 est représenté à la figure 1.

Le système implantable 10 comporte une ancre 15 et un dispositif implantable 20.

Il est entendu par « système implantable » qu'au moins un élément parmi la liste formée de l'ancre 15 et du dispositif implantable 20 est prévu pour être implanté dans le corps humain.

En particulier, il est entendu par « implantable » qu'au moins un élément parmi l'ancre 15 et le dispositif implantable 20 est prévu pour séjourner dans le corps d'un patient P pendant une durée strictement supérieure à une semaine, de préférence strictement supérieure à un mois, de préférence supérieure ou égale à un an.

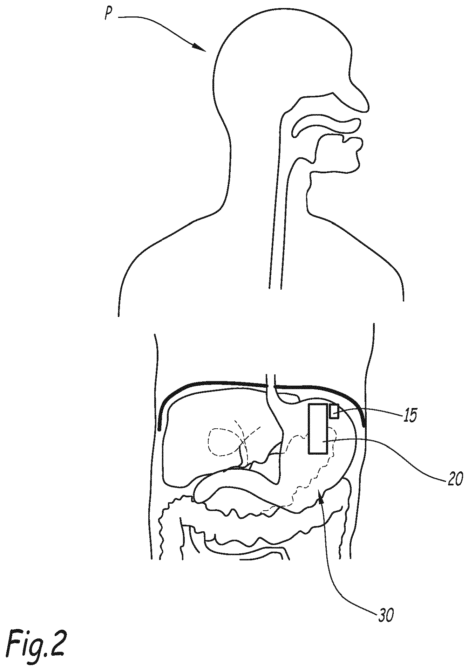

Le système implantable 10 a été représenté schématiquement sur la figure 2 lorsque le système implantable 10 est implanté dans le corps du patient P.

Selon l'exemple de la figure 2, l'ancre 15 et le dispositif implantable 20 sont chacun implanté dans le corps du patient P.

L'ancre 15 est propre à être fixée dans une position prédéterminée dans l'estomac 30 du patient P.

Par exemple, l'ancre 15 est configurée pour être fixée dans la partie supérieure de l'estomac 30. En particulier, l'ancre 15 est configurée pour être fixée dans le fundus gastrique de l'estomac 30. Par exemple, l'ancre 15 est prévue pour être fixée le plus près possible de l'angle de Hiss dans le fundus gastrique.

En variante, l'ancre 15 est configurée pour être fixée dans la partie inférieure de l'estomac 30.

L'ancre 15 est configurée pour supporter le dispositif implantable 20, de préférence de manière amovible. En particulier, l'ancre 15 et le dispositif implantable 20 sont configurés pour être fixés l'un à l'autre, par un dispositif de fixation, et l'ancre 15 est configurée pour maintenir le dispositif implantable 20 dans une position de fixation lorsque l'ancre 15 est fixée dans l'estomac 30.

L'ancre 15 comporte une tête 35 et un premier connecteur 40.

La tête 35 est configurée pour ancrer l'ancre 15 dans la position prédéterminée. En particulier, la tête 35 est configurée pour ancrer l'ancre 15 à la paroi de l'estomac 30.

La tête 35 est, par exemple, un clip gastro-intestinal configuré pour enserrer entre deux branches de la tête 35 une portion de la paroi de l'estomac 30.

En variante, la tête 35 est propre à être suturée par un fil à la paroi de l'estomac 30.

Selon une autre variante, la tête 35 est propre à être enfouie à l'intérieur de la muqueuse gastrique après que celle-ci ait été disséquée.

Le premier connecteur 40 est configuré pour fixer le dispositif implantable 20 à la tête 35.

Le dispositif implantable 20 est configuré pour administrer une substance active au patient P.

Par exemple, le dispositif implantable 20 est configuré pour injecter une substance active dans un fluide corporel du patient P. Le fluide corporel est, par exemple, le liquide péritonéal du patient (voie parentérale). En variante, le fluide corporel est le sang du patient P.

En variante, le dispositif implantable 20 est configuré pour administrer la substance active par la voie entérale, notamment par libération dans l'estomac.

Il est entendu par « substance active » une substance ayant un effet favorable sur la santé du patient.

Par exemple, un médicament est une substance active. Une hormone est un autre exemple de substance active.

La substance active est, par exemple, l'insuline.

En variante, la substance active est un antidouleur. Par exemple, la substance active est la morphine.

Selon une autre variante, la substance active est la levodopa. La levodopa, ou L-dopa, est un médicament utilisé dans le traitement de la maladie de Parkinson.

Selon une autre variante, la substance active est un médicament anticancéreux. Par exemple, la substance active est un composant d'une chimiothérapie.

Le dispositif implantable 20 comporte un premier contrôleur 45, un deuxième connecteur 50, une alimentation électrique 55, un boîtier 65, une réserve de substance active 67 et un injecteur 70.

Le premier contrôleur 45 est une unité de traitement d'informations. Le premier contrôleur 45 comporte une première mémoire 75 et un premier processeur 80.

En variante, le premier contrôleur 45 est réalisé sous forme de circuit intégré dédié, ou encore de composants logiques programmables.

Selon un mode de réalisation, le premier contrôleur 45 comporte un émetteur/récepteur radiofréquence.

Un programme d'ordinateur contenant des instructions de programme est mémorisé dans la première mémoire 75. Les instructions de programme sont propres, lorsqu'elles sont exécutées par le premier processeur 80, à mettre en oeuvre un procédé d'administration de la substance active au patient P.

Le premier processeur 80 est propre à manipuler et/ou transformer des données représentées comme des quantités électroniques ou physiques dans la première mémoire 75 en d'autres données similaires correspondant à des données physiques dans la première mémoire 75, dans des registres ou d'autres types de dispositifs d'affichage, de transmission ou de mémorisation.

Le deuxième connecteur 50 est configuré pour coopérer avec le premier connecteur 40 pour maintenir le dispositif implantable 20 dans la position de fixation.

Par exemple, le deuxième connecteur 50 est configuré pour coopérer avec le premier connecteur 40 par encliquetage.

En variante, le deuxième connecteur 50 comporte un aimant configuré pour fixer le deuxième connecteur au premier connecteur. L'aimant est, par exemple, un électroaimant.

Selon une autre variante, le premier connecteur 40 est configuré pour être solidarisé au deuxième connecteur 50 par vissage. En variante, le premier connecteur 40 comporte une ou de préférence deux baïonnettes complémentaires d'orifices de fixation ménagés dans le deuxième connecteur 50.

De préférence, le deuxième connecteur 50 est prévu pour que le dispositif implantable 20 soit séparable de l'ancre 15. En particulier, le deuxième connecteur 50 est configuré pour que le dispositif implantable 20 soit séparable de l'ancre 15 lorsque l'ancre 15 est fixée dans l'estomac 30 du patient P.

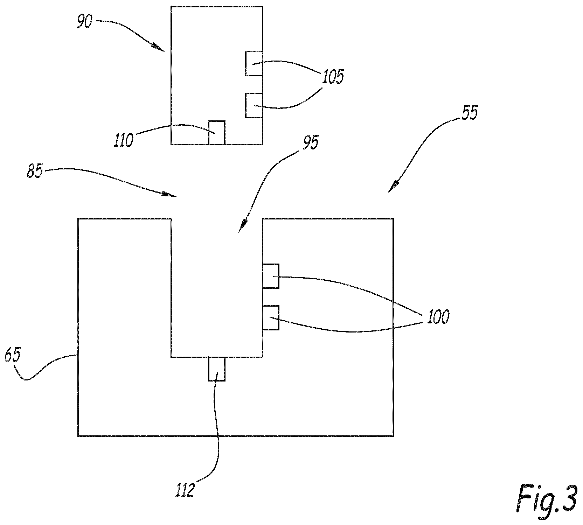

L'alimentation électrique 55 a été représentée sur la figure 3.

L'alimentation électrique 55 est configurée pour alimenter le premier contrôleur 45 avec un premier courant d'alimentation C1.

L'alimentation électrique 55 est, en outre, configurée pour alimenter l'injecteur 70 avec un deuxième courant d'alimentation C2.

L'alimentation électrique 55 comporte un troisième connecteur 85 et une première réserve d'énergie électrique 90.

Le troisième connecteur 85 est configuré pour recevoir de la première réserve d'énergie électrique 90 le premier courant d'alimentation C1 et le deuxième courant d'alimentation C2 et pour alimenter le premier contrôleur 45 et l'injecteur 70 avec, respectivement, le premier courant d'alimentation C1 et le deuxième courant d'alimentation C2.

Le troisième connecteur 85 est configuré pour accueillir la première réserve d'énergie électrique 90. En particulier, le troisième connecteur 85 délimite une cavité 95 configurée pour accueillir au moins partiellement la première réserve d'énergie électrique 90 dans une position de connexion.

Selon l'exemple de la figure 3, la cavité 95 débouche sur l'extérieur du boîtier 65. En particulier, la cavité 95 est configurée pour permettre l'insertion de la première réserve d'énergie électrique 90, depuis l'extérieur du boîtier 65, dans la cavité 95.

Le troisième connecteur 85 comporte, en outre, deux premiers contacts électriques 100, configurés pour être connectés électriquement à la première réserve d'énergie électrique 90 lorsque la première réserve d'énergie électrique 90 est dans la position de connexion. En particulier, les deux premiers contacts électriques 100 débouchent à l'intérieur de la cavité 95.

La première réserve d'énergie électrique 90 est configurée pour stocker de l'énergie électrique. En particulier, la première réserve d'énergie électrique 90 est configurée pour être chargée en énergie électrique à l'extérieur du corps du patient P et pour se décharger lorsque la première réserve d'énergie électrique 90 est dans la position de connexion. Par exemple, la première réserve d'énergie électrique 90 comporte une batterie. En variante, la première réserve d'énergie électrique 90 comporte au moins un capaciteur ou une supercapacité.

La première réserve d'énergie électrique est configurée pour alimenter le premier contrôleur 45 avec le premier courant d'alimentation C1 lorsque la première réserve d'énergie électrique 90 est dans la position de connexion. En outre, la première réserve d'énergie électrique 90 est configurée pour alimenter l'injecteur 70 avec le deuxième courant d'alimentation C2 lorsque la première réserve d'énergie électrique 90 est dans la position de connexion.

Selon l'exemple de la figure 3, la première réserve d'énergie électrique 90 comporte deux deuxièmes contacts électriques 105 complémentaires des premiers contacts électriques 100.

La première réserve d'énergie électrique 90 peut être prévue pour être avalée par le patient P.

Selon une variante, la première réserve d'énergie 90 est propre à être remplacée par endoscopie.

En particulier, la première réserve d'énergie électrique 90 présente un volume strictement inférieur à 6 millilitres (ml).

La première réserve d'énergie électrique 90 présente, en outre, trois dimensions mesurées chacune selon une direction respective, chaque direction étant perpendiculaire aux deux autres directions, et chaque dimension est strictement inférieure à 5 centimètres (cm).

La première réserve d'énergie électrique 90 est mobile entre la position de connexion et une position de déconnexion. Lorsque la première réserve d'énergie électrique 90 est dans la position de déconnexion, la première réserve d'énergie électrique 90 est accueillie dans l'estomac 30 du patient P mais n'est pas connectée électriquement au troisième connecteur 85. Par exemple, lorsque la première réserve d'énergie électrique 90 est dans la position de déconnexion, la première réserve d'énergie électrique est totalement extraite de la cavité 95.

La première réserve d'énergie électrique 90 est configurée pour se déplacer spontanément depuis la position de déconnexion vers la position de connexion. Par exemple, la première réserve d'énergie électrique 90 comporte des attracteurs 110.

Les attracteurs 110 sont configurés pour exercer sur la première réserve d'énergie électrique 90, lorsque la première réserve d'énergie électrique 90 est dans la position de déconnexion, une force tendant à déplacer la première réserve d'énergie électrique 90 depuis la position de déconnexion vers la position de connexion.

En outre, les attracteurs 110 sont configurés pour maintenir la première réserve d'énergie électrique 90 dans la position de connexion.

Les attracteurs 110 comportent, par exemple, un premier aimant propre à coopérer avec un deuxième aimant 112 du troisième connecteur 85. En variante, le premier aimant est propre à coopérer avec une portion ferromagnétique du troisième connecteur 85. Le premier aimant et le deuxième aimant 112 sont, par exemple, des électro-aimants.

Le boîtier 65 est configuré pour isoler le premier contrôleur 45 de l'extérieur du boîtier 65. Par exemple, le boîtier 65 délimite une chambre accueillant au moins le premier contrôleur 45 et l'injecteur 70.

La réserve de substance active 67 est configurée pour stocker la substance active et pour transmettre la substance active à l'injecteur 70.

La réserve de substance active 67 comporte, par exemple, un connecteur solidaire du boîtier 65 et une capsule contenant la substance active. La capsule est propre à être avalée par le patient P et à venir se connecter au connecteur pour délivrer la substance active à l'injecteur. En particulier, chaque capsule est configurée pour éjecter du connecteur correspondant une capsule vide.

L'injecteur 70 est propre à injecter la substance active dans un organe C du patient P. Par exemple, l'injecteur 70 comporte une pompe et un premier cathéter 114.

La pompe est configurée pour prélever la substance active dans la réserve de substance active 67 et pour transmettre la substance active au premier cathéter 114.

Une pompe péristaltique est un exemple de pompe susceptible d'être intégrée à l'injecteur 70.

Selon un exemple, non couvert par les revendications, le dispositif ne comprend qu'un seul premier cathéter. Le premier cathéter 114 présente une première extrémité et une deuxième extrémité. La première extrémité est connectée à la pompe. La deuxième extrémité est propre à administrer au patient P la substance active reçue de la pompe.

Lorsque le fluide corporel F est le liquide péritonéal, la deuxième extrémité débouche dans le péritoine, et l'injecteur 70 est propre à injecter la substance active dans le liquide péritonéal du patient P. La substance active peut passer du liquide péritonéal au système sanguin du patient P.

En variante, lorsque le fluide corporel F est le sang, la deuxième extrémité débouche dans un vaisseau sanguin (veine ou artère) du patient P.

Dans la variante entérale, la deuxième extrémité débouche dans le tube digestif du patient P. Par exemple, la deuxième extrémité débouche dans une lumière de l'intestin du patient P. En variante, la deuxième extrémité débouche dans l'estomac du patient P.

Le fonctionnement du système implantable 10 va maintenant être décrit.

Au cours d'une première étape préalable à l'implantation de l'ancre 15, du dispositif implantable 20 et du deuxième dispositif 25 dans le corps du patient P, la première réserve d'énergie électrique 90 est chargée en énergie électrique. La première réserve d'énergie électrique 90 génère donc le premier courant d'alimentation C1 à destination du premier contrôleur 45.

Au cours d'une deuxième étape, l'ancre 15, le dispositif implantable 20 et le deuxième dispositif 25 sont implantés dans le corps du patient P.

Au cours d'une troisième étape, un message d'activation est transmis, par un dispositif externe, au dispositif implantable 20. En particulier, le message d'activation est transmis par communication radiofréquence. Le message d'activation informe le premier contrôleur 45 que le système implantable 10 a bien été implanté dans le corps du patient P.

Au cours d'une quatrième étape, la substance active est administrée au patient P.

Le premier contrôleur 45 commande l'administration d'une quantité de substance active au patient P, par l'injecteur 70, avec une fréquence d'administration.

Par exemple, la quantité de substance active administrée et la fréquence d'administration sont prédéterminées.

Le dispositif implantable 20 permet de délivrer des substances actives, soit par voie entérale, soit par voie parentérale. Le dispositif implantable 20 permet donc de délivrer de manière simple et non douloureuse pour le patient P des substances actives qui sont habituellement injectées, par exemple par voie intraveineuse ou intramusculaire.

Le dispositif implantable 20 permet également de délivrer de manière automatique et régulière des substances actives, par exemple dans le cas où l'arrivée dans l'intestin doit impérativement se faire à un débit aussi constant que possible, telle que la L-Dopa pour certains patients Parkinsoniens. Grâce au dispositif 20, l'administration de la substance active est facilitée. En effet, la capsule contenant la substance active peut être administrée au patient par voie orale, ce qui est donc peu douloureux et peu contraignant pour le patient. En particulier, le nombre de capsules de substance active à avaler est bien inférieur au nombre de prises de médicament à effectuer, sur une même période, pour arriver au même débit d'administration si les médicaments sont administrés sous forme de pilules classiques.

En outre, le dispositif implantable 20, une fois qu'il est installé, est peu contraignant pour le patient P. En particulier, le dispositif implantable 20 est susceptible de remplacer des perfusions externes, mais ne constitue pas comme celles-ci une entrave au déplacement du patient.

Du fait que le dispositif implantable 20 est dans l'estomac, le remplacement de la première réserve d'énergie électrique 90 est aisé et peut, par exemple, être effectué par voie endoscopique depuis l'oesophage, de manière simple et rapide. En outre, le remplacement de la première réserve d'énergie électrique 90 pose peu de risques d'infection puisque aucune incision n'est réalisée.

L'utilisation des attracteurs 110 rend encore plus simple la mise en place de la première réserve d'énergie électrique 90, même sans endoscopie, puisqu'il suffit alors que le patient P avale la première réserve d'énergie électrique 90.

En outre, le système implantable 10 ne suppose pas que le patient P porte en permanence des moyens de stockage d'énergie électrique à l'extérieur de son corps, ni que des conducteurs électriques disgracieux débouchent hors du corps du patient P. Le système implantable 10 est donc peu contraignant pour le patient.

Un deuxième exemple de système implantable 10, non couvert par les revendications, va maintenant être décrit. Les éléments identiques au premier exemple de système implantable 10 de la figure 1 ne sont pas décrits à nouveau. Seules les différences sont mises en évidence.

Le dispositif implantable 20 comporte au moins un capteur. Chaque capteur est propre à mesurer une valeur d'un paramètre représentatif d'un phénomène physiologique du patient P.

Un paramètre est, par exemple, un taux d'un marqueur biologique dans un fluide corporel F du patient P.

Le dispositif implantable 20 comporte alors un deuxième cathéter configuré pour conduire un fluide corporel F du patient P depuis l'organe C jusqu'au capteur. Par exemple, l'organe C est le péritoine, et le fluide F est le fluide péritonéal.

Le marqueur biologique est, par exemple, le glucose.

En variante, le marqueur biologique est un ion présent dans le fluide corporel F.

Le capteur est alors propre à mesurer un taux du marqueur biologique dans le fluide corporel F. Par exemple, le capteur est propre à mesurer un taux de glucose.

Le premier contrôleur 45 est configuré pour commander l'injection de la substance active, par l'injecteur 70, en fonction d'au moins une valeur du paramètre mesurée. Le premier contrôleur 45 est propre à modifier une période ou une fréquence d'administration de la substance active et/ou une dose de substance active à administrer au patient en fonction des valeurs mesurées.

Par exemple, le capteur mesure des valeurs du paramètre avec une période de mesure prédéterminée, et le premier contrôleur 45 commande l'administration de la substance active, par l'injecteur 70, en fonction des valeurs mesurées.

Par exemple, le premier contrôleur 45 compare les valeurs du paramètre mesuré à un seuil prédéterminé et commande l'administration d'une dose de substance active si une ou plusieurs valeurs mesurées sont supérieures ou égales au seuil prédéterminé. La dose de substance active administrée est, par exemple, une dose correspondant à un volume prédéterminé.

En variante, le premier contrôleur 45 commande l'administration d'une dose de substance active avec une période d'administration prédéterminée, chaque dose administrée correspondant à un volume de substance active calculé en fonction des valeurs mesurées.

Selon une variante du deuxième exemple, le capteur est séparé du dispositif implantable 20. Par exemple, le système implantable 10 comprend un dispositif additionnel propre à être implanté dans le corps du patient P et à communiquer par communication radiofréquence avec le dispositif implantable 20.

Le capteur est alors intégré au dispositif additionnel. Par exemple, le dispositif additionnel est configuré pour transmettre les valeurs mesurées au dispositif implantable 20 par communication radiofréquence.

Dans ce cas, le dispositif additionnel comprend un deuxième contrôleur comportant une deuxième mémoire et un deuxième processeur et propre à commander l'acquisition de valeurs du paramètre par le capteur ainsi que l'émission de messages radiofréquence de transmission des valeurs mesurées au dispositif implantable 20.

Un troisième exemple de système implantable 10, non couvert par les revendications, va maintenant être décrit. Les éléments identiques au premier exemple de système implantable 10 ne sont pas décrits à nouveau. Seules les différences sont mises en évidence.

L'alimentation électrique 55 ne comporte pas de troisième connecteur 85 ni de réserve d'énergie électrique 90.

L'alimentation électrique 55 comporte un générateur d'énergie électrique. Il est entendu par « générateur d'énergie électrique » que le générateur d'énergie électrique n'est pas configuré pour être chargé en énergie électrique par un courant électrique.

Le générateur d'énergie électrique est propre à générer au moins un courant électrique par réaction d'au moins une espèce chimique présente dans le corps du patient P. Plus précisément, le générateur d'énergie électrique est propre à générer le premier courant d'alimentation C1 et le deuxième courant d'alimentation C2.

Par exemple, le générateur d'énergie électrique comprend deux électrodes, les électrodes baignant dans les sucs gastriques du patient P lorsque le dispositif de centralisation 20 est dans la position de fixation. En variante, les électrodes du générateur d'énergie électrique sont prévues pour baigner dans l'intestin du patient P lorsque le dispositif de centralisation 20 est dans la position de fixation.

Chaque électrode comporte au moins une enzyme. En variante, chaque électrode comporte au moins un micro-organisme. Par exemple, chaque électrode du générateur d'énergie électrique comporte un conducteur électrique recouvert de l'enzyme ou du micro-organisme, l'ensemble ainsi formé étant entouré d'une membrane. La membrane est, par exemple, configurée pour être traversée par certaines espèces chimiques naturellement présentes dans l'estomac ou l'intestin du patient P.

Lorsque les électrodes du générateur d'énergie électrique sont immergées dans les sucs gastriques ou dans le liquide intestinal, l'une des électrodes joue le rôle d'anode dans une réaction d'oxydo-réduction mettant en jeu une première espèce chimique. Simultanément, l'autre électrode joue le rôle de cathode dans une réaction d'oxydoréduction mettant en jeu une deuxième espèce chimique.

Par l'oxydation et la réduction simultanée de la première espèce chimique et de la deuxième espèce chimique, une tension électrique apparaît entre les deux conducteurs électriques. Le premier courant d'alimentation C1 et le deuxième courant d'alimentation C2 sont alors générés.

La première espèce chimique est, par exemple, le glucose. La deuxième espèce chimique est, par exemple, l'oxygène.

La première espère chimique est, par exemple, le glucose. La deuxième espèce chimique est, par exemple, l'oxygène.

Le troisième exemple de système implantable 10 ne nécessite pas de charger électriquement une réserve d'énergie électrique 90 ni de faire pénétrer la réserve d'énergie électrique 90 dans le corps du patient P.

Les contraintes pour le patient P sont, là encore, réduites.

Selon un quatrième exemple, non couvert par les revendications, le générateur d'énergie électrique est propre à générer au moins un courant électrique par conversion d'une énergie mécanique en énergie électrique. En particulier, le générateur d'énergie électrique est propre à générer au moins un courant électrique à partir des mouvements de l'estomac 30.

Dans la description ci-dessus, les fonctions du système implantable 10 ont été séparées en plusieurs exemples pour faciliter leur compréhension par le lecteur. Cependant, il est à noter que les exemples précédents peuvent être combinés pour générer de nouveaux modes de réalisation.

De plus, la description ci-dessus a été donnée dans le cas où l'ancre 15 et le dispositif implantable 20 forment deux dispositifs séparés. L'homme du métier comprendra aisément que le dispositif implantable 20 et l'ancre 15 sont susceptibles de former un seul dispositif, l'ancre 15 et le dispositif implantable 20 n'étant alors pas séparables l'un de l'autre. Par exemple, l'ancre 15 est venue de matière avec le boîtier 65 du dispositif implantable 20.

Un cinquième exemple de système implantable 10 va maintenant être décrit. Les éléments identiques au premier exemple de système implantable 10 ne sont pas décrits à nouveau. L'injecteur 70 comporte deux premiers cathéters 114.

La pompe est configurée pour aspirer à travers un premier cathéter 114 un fluide corporel F, et pour rejeter par l'autre cathéter 114 le fluide corporel F aspiré. Selon un mode de réalisation, la pompe est, en outre, configurée pour injecter la substance active dans le fluide corporel F de manière que le fluide F rejeté soit chargé en la substance active.

Selon un mode de réalisation, chaque premier cathéter 114 traverse la paroi de l'estomac 30 pour déboucher entre le feuillet pariétal et le feuillet viscéral du péritoine. En particulier, la deuxième extrémité de chaque premier cathéter 114 débouche entre le feuillet pariétal et le feuillet viscéral du péritoine.

La longueur et/ou le diamètre des premiers cathéters selon l'invention est généralement adaptée à la situation du lieu de captation du liquide corporel et/ou de refoulement de ce liquide après passage dans la pompe. Lorsque le fluide corporel F est le liquide péritonéal, par exemple (mais pas exclusivement) chaque premier cathéter 114 peut notamment présenter une longueur comprise entre 1 centimètre (cm) et 20 cm, par exemple entre 2 cm et 15 cm, en particulier entre 6 cm et 12 cm. Chaque premier cathéter 114 peut présenter un diamètre inférieur ou égal à 3 millimètres (mm), par exemple inférieur ou égal à 2 mm.

Selon un mode de réalisation, lorsque le dispositif implantable 20 comporte deux premiers cathéters 114, l'un des premiers cathéters 114 remplit le rôle de deuxième cathéter.

Un procédé d'administration mis en oeuvre à l'aide d'un dispositif implantable 20 conforme au cinquième exemple va maintenant être décrit.

Le procédé d'administration comporte une étape d'aspiration, une étape d'injection et une étape d'expulsion.

Lors de l'étape d'aspiration, un flux de fluide corporel F est aspiré par l'un des premiers cathéters 114. Par exemple, le premier contrôleur 45 commande l'aspiration du fluide corporel F par la pompe.

Lors de l'étape d'injection, une dose de la substance active est injectée par la pompe dans le flux de fluide corporel F. Par exemple, le premier contrôleur 45 commande l'injection par la pompe de la substance active dans le flux de fluide corporel F.

Lors de l'étape d'expulsion, le fluide corporel F contenant la substance active est rejeté par l'autre premier cathéter 114. En particulier, le fluide corporel F contenant la substance active est rejeté dans le même organe ou la même cavité du patient P d'où le fluide corporel F a été aspiré. La substance active est donc ainsi administrée dans le fluide corporel F du patient P.

Selon un mode de réalisation, les deux premiers cathéters 114 débouchent dans le péritoine, par exemple entre le feuillet viscéral et le feuillet pariétal du péritoine. Le fluide corporel F est alors le liquide péritonéal.

Il est à noter que les deux premiers cathéters 114 sont susceptibles de déboucher dans d'autres organes du patient P. Par exemple, les deux premiers cathéters 114 débouchent chacun dans un vaisseau sanguin du patient P. Le fluide corporel F est alors le sang.

Les étapes d'aspiration, d'injection et d'expulsion ont été décrites ci-dessus comme des étapes séparées. Cependant, ces trois étapes sont susceptibles d'être mises en oeuvre simultanément. Par exemple, un flux continu de fluide corporel F est aspiré par l'un des premiers cathéters 114, reçoit la substance active et est rejeté par l'autre premier cathéter 114.

Selon un mode de mise en oeuvre, les étapes d'aspiration, d'injection et d'expulsion sont mises en oeuvre de manière continue sur une période de temps prolongée, par exemple une période de temps supérieure ou égale à une heure, supérieure ou égale à une journée, ou encore supérieure ou égale à une semaine.

Selon un autre mode de mise en oeuvre, les étapes d'aspiration, d'injection et d'expulsion sont mises en oeuvre de manière fractionnée, en particulier avec une fréquence déterminée par le premier contrôleur 45. La fréquence est, par exemple, déterminée par le premier contrôleur 45 à partir des valeurs du paramètre mesuré par le capteur.

Lors d'une étape optionnelle de nettoyage, un flux de fluide corporel F est aspiré par l'un des premiers cathéters 114 et rejeté par l'autre premier cathéter 114 sans qu'une substance active soit injectée dans le fluide corporel F. Le flux de fluide corporel expulse alors du premier cathéter 114 par lequel le flux est rejeté des particules, agglomérats ou autres éléments solides, qui auraient pénétré, ou se seraient formés, dans la lumière du premier cathéter 114. Cette étape est, par exemple, répétée ensuite en inversant le premier cathéter 114 par lequel le flux est rejeté et le premier cathéter 114 par lequel le flux est aspiré.

Selon une variante, l'étape de nettoyage comprend l'injection au travers de chaque premier cathéter 114 d'une enzyme propre à dissoudre des formations fibrineuses ou des caillots qui se seraient formés dans le ou les cathéters 114. L'urokinase et la streptokinase sont des exemples d'enzymes.

L'injection d'enzyme est, par exemple, réalisée au moins une fois par mois, par exemple au moins une fois par semaine. Selon un mode de réalisation, l'injection d'enzyme est réalisée une fois par jour.

L'enzyme est, en particulier, stockée dans une réserve d'enzyme intégrée au dispositif implantable 20.

Lorsque l'injection de substance active a lieu via un flux de fluide corporel F aspiré par un premier cathéter 114 et rejeté par l'autre premier cathéter 114, le rejet de la substance active hors du premier cathéter 114 est facilité par le flux de fluide corporel F. En particulier, la totalité de la substance active injectée par la pompe est efficacement rejetée hors du premier cathéter 114 même si la quantité de substance active est très faible et risquerait sinon de rester partiellement à l'intérieur du premier cathéter, ou si le fluide corporel F dans lequel la substance active est rejetée exerce une pression susceptible d'empêcher la sortie de la substance active hors du premier cathéter 114.

La régulation de l'injection est donc plus efficace, puisque de très petites quantités de substance active peuvent être administrées au patient avec une grande précision. En particulier, il est envisageable de répartir temporellement l'administration, en administrant de très faibles doses de substance active à de multiples reprises ou suivant une libération continue sur une durée déterminée. Cette disposition de l'invention permet en effet une dilution régulée avantageuse de la substance active.

Les doses de substance active administrées étant très faibles, les effets secondaires pour le patient P sont susceptibles d'être réduits par rapport à une administration de doses plus importantes avec une fréquence plus faible.

En outre, le flux de fluide corporel F rejeté par le premier cathéter 114 permet de déboucher un premier cathéter 114 qui serait bouché partiellement ou totalement, lors de l'étape d'injection de substance active ou d'une étape de nettoyage.

Chaque premier cathéter 114 est réalisé en un matériau biocompatible. Le matériau biocompatible est par exemple choisi parmi les polyuréthanes, le silicone, les nylons, les résines polycarbonates ou les polysulfones.

Chaque premier cathéter 114 est, par exemple, réalisé en un matériau poreux. En particulier, le matériau poreux est configuré pour être traversé au moins par la substance active. Selon un mode de réalisation, le matériau poreux est configuré pour être traversé par la substance active et par le fluide corporel F.

Le polyvinylalcool (également appelé PVA) est un exemple de matériau poreux adapté pour former un premier cathéter 114.

Selon une variante, le matériau poreux est un matériau naturellement non-poreux mais dans lequel des perforations ont été ménagées. Les perforations jouent alors le rôle de pores.

Des modes de réalisation dans lesquels la deuxième extrémité est ouverte, c'est-à-dire où la lumière centrale du premier cathéter 114 débouche à l'extérieur du premier cathéter 114 sont envisageables, tout comme des modes de réalisation dans lesquels la deuxième extrémité est fermée.

Lorsque le ou les premiers cathéters 114 sont réalisés en un matériau poreux, le fluide corporel F est aspiré en partie à travers la deuxième extrémité et en partie à travers les pores ou perforations du matériau poreux. De même, la substance active, et le cas échéant le fluide corporel F aspiré sont rejetés en partie à travers la deuxième extrémité et en partie à travers les pores ou perforations du matériau poreux.

Lorsque la deuxième extrémité de chaque premier cathéter 114 est fermée, la substance active, et le cas échéant le fluide corporel F, sont échangés entre le premier cathéter 114 et le corps du patient à travers les pores ou perforations du premier cathéter 114.

Le choix d'un matériau poreux pour le ou les premiers cathéters 114 permet une aspiration et/ou une expulsion de liquide à travers les pores, sur toute la longueur du premier cathéter 114 concerné. Le ou les premiers cathéters 114 sont alors moins sensibles à une éventuelle obstruction de la lumière du cathéter. En outre, la substance active est répartie dans une plus grande zone du corps du patient P que si la substance active était expulsée uniquement à travers la deuxième extrémité. D'éventuels effets secondaires dépendant de la densité volumique de substance active sont donc limités.

Selon un mode de réalisation, chaque premier cathéter 114 comporte un élément d'appui propre à venir en appui contre la paroi externe de l'estomac 30 ou contre le feuillet viscéral du péritoine. L'élément d'appui est, par exemple, configuré pour, lorsqu'il est en appui contre la paroi externe de l'estomac 30 ou contre le feuillet viscéral du péritoine, exercer une force tendant à rapprocher le dispositif implantable 20 de la paroi intérieure de l'estomac 30, en particulier à plaquer le dispositif implantable 20 contre la paroi intérieure de l'estomac 30.

Dans cet exemple, le ou les premier(s) cathéter(s) 114 joue(nt) le rôle d'ancre 15 pour fixer le dispositif implantable 20 à la paroi de l'estomac 30.

Selon une variante, chaque premier cathéter 114 est fixé à la paroi stomacale grâce à des points de suture traversant conjointement la paroi de l'estomac 30 et le premier cathéter 114. Dans ce cas, chaque premier cathéter 114 porte, par exemple, au moins un fil ou lien susceptible d'être fixé à la paroi de l'estomac 30.

Selon un autre exemple, le dispositif implantable 20 est au moins partiellement intégré à l'intérieur de la muqueuse stomacale. Par exemple, le dispositif implantable 20 est placé entre la muqueuse stomacale et la musculeuse stomacale. Selon un mode de mise en place, le dispositif implantable 20 est placé entre la muqueuse stomacale et la sous-muqueuse stomacale.

Selon encore un exemple, la tête 35 comporte au moins une semelle située à l'extérieur de l'estomac 30. Par exemple, la tête 35 comporte deux semelles.

Chaque semelle est configurée pour venir en appui contre la face extérieure de la paroi de l'estomac 30 et pour être reliée au dispositif implantable 20 de manière à exercer une force tendant à plaquer le dispositif implantable 20 contre la face intérieure de la paroi de l'estomac 30. Selon une variante, chaque semelle est configurée pour être placée entre le feuillet viscéral et le feuillet pariétal du péritoine et pour venir en appui contre le feuillet viscéral pour plaquer le dispositif implantable 20 contre la face intérieure de la paroi de l'estomac 30.

Chaque semelle est, par exemple, une plaque. En variante, chaque semelle comporte un treillis de fils tendus sur un cadre, en particulier un cadre souple propre à être plié et inséré dans un endoscope ou une aiguille creuse.

Le premier connecteur 40 comporte, par exemple, un ou plusieurs anneaux solidaires du boîtier 65. Chaque semelle est, par exemple, fixée au dispositif implantable 20 par un ou plusieurs fils fixés à un ou plusieurs des anneaux.

La fixation par une ou plusieurs semelles permet de répartir la pression exercée par le dispositif implantable sur une plus grande surface de la paroi de l'estomac 30 et de diminuer donc la force ainsi exercée. En outre, ce mode de fixation ne suppose pas de générer dans la paroi stomacale un pli qui réduit le volume de l'estomac, qui est susceptible de générer des tensions dans l'ancre qui y est fixée. Puisque les forces exercées sur la paroi stomacale sont réduites, les risques d'apparition d'une réaction inflammatoire de la muqueuse gastrique sont limités.

## Revendications

1. Dispositif implantable (20) propre à être fixé dans une position de fixation à une paroi de l'estomac (30) d'un patient (P), le dispositif implantable (20) étant accueilli dans l'estomac (30) lorsque le dispositif implantable (20) est dans la position de fixation, le dispositif implantable comprenant une réserve (67) d'une substance active et un injecteur (70) propre à administrer la substance active au patient (P), le dispositif implantable (20) étant **caractérisé en ce que** l'injecteur (70) comporte deux cathéters (114), l'injecteur (70) étant configuré pour administrer la substance active dans un fluide corporel du patient (P), à travers au moins un des cathéters (114), l'injecteur (70) étant configuré pour aspirer, par l'un des cathéters (114) le fluide corporel (F) du patient (P) et pour rejeter par l'autre cathéter (114) le fluide corporel (F) aspiré.

2. Dispositif implantable (20) selon la revendication 1, dans lequel le fluide corporel est le liquide péritonéal ou le sang.

3. Dispositif implantable (20) selon la revendication 1, dans lequel l'injecteur (70) est configuré pour injecter la substance active dans le tube digestif du patient (P).

4. Dispositif implantable (20) selon la revendication 3, dans lequel un cathéter (114) est propre à permettre l'injection de la substance active, par l'injecteur (70), dans une lumière de l'intestin du patient (P).

5. Dispositif implantable (20) selon l'une quelconque des revendications 1 à 4, dans lequel la substance active est choisie parmi l'ensemble formé de : l'insuline, la levodopa, un antidouleur et un anticancéreux.

6. Dispositif implantable (20) selon l'une quelconque des revendications 1 à 5, dans lequel le dispositif implantable (20) comprend un capteur et un cathéter propre à conduire un fluide corporel (F) du patient (P) au capteur, le capteur étant propre à mesurer au moins une valeur d'un taux d'un marqueur biologique dans le fluide corporel (F).

7. Dispositif implantable (20) selon la revendication 6, comprenant en outre un contrôleur (45) propre à commander l'administration, par l'injecteur (70), de la substance active en fonction d'au moins une valeur du taux mesurée.

8. Dispositif implantable (20) selon la revendication 7, dans lequel le contrôleur (45) est propre à calculer une fréquence et/ou une dose de substance active à administrer au patient (P) en fonction d'au moins une valeur du taux mesurée.

9. Dispositif implantable (20) selon l'une quelconque des revendications 1 à 8, dans lequel le dispositif implantable (20) comprend une alimentation électrique (55) comportant une réserve (90) d'énergie électrique amovible et un connecteur (85) propre à accueillir la réserve d'énergie électrique (90), le connecteur (85) comprenant deux premiers contacts électriques (100), la réserve d'énergie électrique (90) étant propre à alimenter électriquement l'injecteur (70) lorsque la réserve d'énergie électrique (90) est connectée électriquement au connecteur (85) dans une position de connexion, la réserve d'énergie électrique comprenant deux deuxièmes contacts électriques (105) complémentaires des deux premiers contacts électriques (100), et de préférence la réserve électrique (90) étant configurée pour être avalée par le patient (P) et pour se déplacer spontanément jusqu'à la position de connexion depuis une position de déconnexion dans laquelle la réserve d'énergie électrique (90) est accueillie dans l'estomac (30) du patient (P) et est déconnectée du connecteur (85).

10. Dispositif implantable (20) selon l'une quelconque des revendications 1 à 8, dans lequel le dispositif implantable (20) comprend une alimentation électrique (55) propre à générer un courant électrique d'alimentation de l'injecteur par réaction d'au moins une espèce chimique présente dans le corps du patient (P), notamment le glucose.

11. Dispositif implantable (20) selon l'une quelconque des revendications 1 à 8, dans lequel le dispositif implantable (20) comprend une alimentation électrique (55) propre à générer un courant électrique d'alimentation de l'injecteur par conversion d'énergie mécanique en énergie électrique.

12. Dispositif implantable (20) selon l'une quelconque des revendications précédentes, dans lequel l'injecteur (70) est configuré pour injecter la substance active dans le fluide corporel (F) rejeté.

13. Dispositif implantable (20) selon la revendication 12, dans lequel l'injecteur est configuré pour rejeter le fluide corporel (F) contenant la substance active dans le même organe ou la même cavité du patient (P) d'où le fluide corporel F a été aspiré.

14. Dispositif implantable (20) selon l'une quelconque des revendications précédentes, dans lequel au moins un cathéter (114) est réalisé en un matériau poreux.

15. Dispositif implantable (20) selon la revendication 14, dans lequel le matériau poreux est propre à être traversé par la substance active et/ou par un fluide corporel (F) du patient (P).

16. Dispositif implantable (20) selon l'une quelconque des revendications précédentes, dans lequel le dispositif implantable (20) est configuré pour être au moins partiellement intégré à l'intérieur de la muqueuse stomacale, entre la muqueuse stomacale et la sous-muqueuse stomacale.

## Patentansprüche

1. Implantierbare Vorrichtung (20), die dazu geeignet ist, in einer Fixierungsposition an einer Magenwand (30) eines Patienten (P) fixiert zu werden, wobei die implantierbare Vorrichtung (20) im Magen (30) untergebracht ist, wenn sich die implantierbare Vorrichtung (20) in der Fixierungsposition befindet, wobei die implantierbare Vorrichtung einen Speicher (67) eines Wirkstoffs und einen Injektor (70) umfasst, der dazu geeignet ist, den Wirkstoff an den Patienten (P) zu verabreichen, wobei die implantierbare Vorrichtung (20) **dadurch gekennzeichnet ist, dass** der Injektor (70) zwei Katheder (114) enthält, wobei der Injektor (70) dazu konfiguriert ist, den Wirkstoff durch mindestens einen der Katheder (114) in eine Körperflüssigkeit des Patienten (P) zu verabreichen, wobei der Injektor (70) dazu konfiguriert ist, durch einen der Katheder (114) die Körperflüssigkeit (F) des Patienten (P) abzusaugen und durch den anderen Katheder (114) die abgesaugte Körperflüssigkeit (F) auszuscheiden.

2. Implantierbare Vorrichtung (20) nach Anspruch 1, wobei die Körperflüssigkeit Peritonealflüssigkeit oder Blut ist.

3. Implantierbare Vorrichtung (20) nach Anspruch 1, wobei der Injektor (70) dazu konfiguriert ist, den Wirkstoff in den Verdauungstrakt des Patienten (P) zu injizieren.

4. Implantierbare Vorrichtung (20) nach Anspruch 3, wobei ein Katheter (114) dazu geeignet ist, die Injektion des Wirkstoffs durch den Injektor (70) in ein Lumen des Darms des Patienten (P) zu ermöglichen.

5. Implantierbare Vorrichtung (20) nach einem der Ansprüche 1 bis 4, wobei der Wirkstoff aus der Gruppe ausgewählt ist, die aus Folgendem besteht: Insulin, Levodopa, einem Schmerzmittel und einem Krebsmedikament.

6. Implantierbare Vorrichtung (20) nach einem der Ansprüche 1 bis 5, wobei die implantierbare Vorrichtung (20) einen Sensor und einen Katheter umfasst, der dazu geeignet ist, eine Körperflüssigkeit (F) des Patienten (P) zum Sensor zu leiten, wobei der Sensor dazu geeignet ist, mindestens einen Wert eines Spiegels eines Biomarkers in der Körperflüssigkeit (F) zu messen.

7. Implantierbare Vorrichtung (20) nach Anspruch 6, die ferner eine Steuerung (45) umfasst, die dazu geeignet ist, die Verabreichung des Wirkstoffs durch den Injektor (70) in Abhängigkeit von mindestens einem gemessenen Wert des Spiegels zu steuern.

8. Implantierbare Vorrichtung (20) nach Anspruch 7, wobei die Steuerung (45) dazu geeignet ist, eine Häufigkeit und/oder eine Dosis des dem Patienten (P) zu verabreichenden Wirkstoffs in Abhängigkeit von mindestens einem gemessenen Wert des Spiegels zu berechnen.

9. Implantierbare Vorrichtung (20) nach einem der Ansprüche 1 bis 8, wobei die implantierbare Vorrichtung (20) eine elektrische Stromversorgung (55) umfasst, die einen entfernbaren elektrischen Energiespeicher (90) und einen Anschluss (85) umfasst, der dazu geeignet ist, den elektrischen Energiespeicher (90) aufzunehmen, wobei der Anschluss (85) zwei erste elektrische Kontakte (100) umfasst, wobei der elektrische Energiespeicher (90) dazu geeignet ist, den Injektor (70) elektrisch mit Strom zu versorgen, wenn der elektrische Energiespeicher (90) mit dem Anschluss (85) in einer Verbindungsposition verbunden ist, wobei der elektrische Energiespeicher zwei zweite elektrische Kontakte (105) umfasst, die komplementär mit den beiden ersten elektrischen Kontakten (100) sind, und wobei der elektrische Speicher (90) vorzugsweise dazu konfiguriert ist, vom Patienten (P) geschluckt zu werden und sich von einer Trennposition, in der der elektrische Energiespeicher (90) im Magen (30) des Patienten (P) untergebracht ist, spontan in die Verbindungsposition zu bewegen, und vom Anschluss (85) getrennt wird.

10. Implantierbare Vorrichtung (20) nach einem der Ansprüche 1 bis 8, wobei die implantierbare Vorrichtung (20) eine elektrische Stromversorgung (55) umfasst, die dazu geeignet ist, einen elektrischen Versorgungsstrom des Injektors durch Reaktion mit mindestens einer im Körper des Patienten (P) vorhandenen chemischen Spezies, insbesondere Glukose, zu erzeugen.

11. Implantierbare Vorrichtung (20) nach einem der Ansprüche 1 bis 8, wobei die implantierbare Vorrichtung (20) eine elektrische Stromversorgung (55) umfasst, die dazu geeignet ist, einen elektrischen Stromversorgungsstrom des Injektors durch Umwandlung mechanischer Energie in elektrische Energie zu erzeugen.

12. Implantierbare Vorrichtung (20) nach einem der vorhergehenden Ansprüche, wobei der Injektor (70) dazu konfiguriert ist, zwei Katheter umfasst, wobei der Injektor dazu konfiguriert ist, den Wirkstoff in die ausgeschiedene Körperflüssigkeit (F) zu injizieren.

13. Implantierbare Vorrichtung (20) nach Anspruch 12, wobei der Injektor dazu konfiguriert ist, die Körperflüssigkeit (F), die den Wirkstoff enthält, in das gleiche Organ oder den gleichen Hohlraum des Patienten (P) auszuscheiden, aus der die Körperflüssigkeit (F) abgesaugt wurde.

14. Implantierbare Vorrichtung (20) nach einem der vorhergehenden Ansprüche, wobei der mindestens eine Katheter (114) aus einem porösen Material hergestellt ist.

15. Implantierbare Vorrichtung (20) nach Anspruch 14, wobei das poröse Material dazu geeignet ist, vom Wirkstoff und/oder von einer Körperflüssigkeit (F) des Patienten (P) durchströmt zu werden.

16. Implantierbare Vorrichtung (20) nach einem der vorhergehenden Ansprüche, wobei die implantierbare Vorrichtung (20) dazu konfiguriert ist, mindestens teilweise innerhalb der Magenschleimhaut, zwischen der Magenschleimhaut und der Magenunterschleimhaut, integriert zu werden.

## Claims

1. An implantable device (20) suitable for being fixed in a fixing position to a wall of the stomach (30) of a patient (P), the implantable device (20) being received in the stomach (30) when the implantable device (20) is in the fixing position, the implantable device comprising a reserve (67) of an active substance and an injector (70) suitable for administering the active substance to the patient (P), the implantable device (20) being **characterised in that** the injector (70) includes two catheters (114), the injector (70) being configured to administer the active substance into a body fluid of the patient (P), through at least one of the catheters (114), the injector (70) being configured to suck, through one of the catheters (114), the body fluid (F) of the patient (P) and to reject, through the other catheter (114), the sucked body fluid (F).

2. The implantable device (20) according to claim 1, wherein the body fluid is peritoneal fluid or blood.

3. The implantable device (20) according to claim 1, wherein the injector (70) is configured to inject the active substance into the digestive tract of the patient (P) .

4. The implantable device (20) according to claim 3, wherein a catheter (114) is capable of allowing the injection of the active substance, by the injector (70), into a lumen of the intestine of the patient (P).

5. The implantable device (20) according to any one of claims 1 to 4, wherein the active substance is selected from the group formed by: insulin, levodopa, a painkiller and an anticancer drug.

6. The implantable device (20) according to any one of claims 1 to 5, wherein the implantable device (20) comprises a sensor and a catheter capable of conducting a body fluid (F) from the patient (P) to the sensor, the sensor being capable of measuring at least one value of a level of a biological marker in the body fluid (F).

7. The implantable device (20) according to claim 6, further comprising a controller (45) capable of controlling the administration, by the injector (70), of the active substance depending on at least one value of the measured level.

8. The implantable device (20) according to claim 7, wherein the controller (45) is capable of calculating a frequency and/or a dose of active substance to be administered to the patient (P) depending on at least one value of the measured level.

9. The implantable device (20) according to any one of claims 1 to 8, wherein the implantable device (20) comprises a power supply (55) including a removable reserve (90) of electrical energy and a connector (85) capable of receiving the reserve of electrical energy (90), the connector (85) comprising two first electrical contacts (100), the reserve of electrical energy (90) being capable of supplying power to the injector (70) when the reserve of electrical energy (90) is electrically connected to the connector (85) in a connection position, the reserve of electrical energy comprising two second electrical contacts (105) complementary to the first two electrical contacts (100), and preferably the reserve of electrical energy (90) being configured to be swallowed by the patient (P) and to move spontaneously to the connection position from a disconnection position in which the reserve of electrical energy (90) is received in the stomach (30) of the patient (P) and is disconnected from the connector (85) .

10. The implantable device (20) according to any one of claims 1 to 8, wherein the implantable device (20) comprises a power supply (55) capable of generating an electric current for powering the injector by reaction of at least one chemical species present in the body of the patient (P), in particular glucose.

11. The implantable device (20) according to any one of claims 1 to 8, wherein the implantable device (20) comprises a power supply (55) capable of generating an electric current for powering the injector by conversion of mechanical energy into electrical energy.

12. The implantable device (20) according to any one of the preceding claims, wherein the injector (70) is configured to inject the active substance into the rejected body fluid (F).

13. The implantable device (20) according to claim 12, wherein the injector is configured to reject the body fluid (F) containing the active substance into the same organ or the same cavity of the patient (P) from which the body fluid F was sucked.

14. The implantable device (20) according to any one of the preceding claims, wherein at least one catheter (114) is made of a porous material.

15. The implantable device (20) according to claim 14, wherein the porous material is capable of being passed through by the active substance and/or by a body fluid (F) of the patient (P).

16. The implantable device (20) according to any one of the preceding claims, wherein the implantable device (20) is configured to be at least partially integrated inside the stomach mucosa, between the stomach mucosa and the stomach submucosa.
